# EUROPÄISCHE PATENTSCHRIFT

(11) **EP 0 261 181 B1**
(45) Veröffentlichungstag und Bekanntmachung des Hinweises auf die Patenterteilung: **25.08.1993**
(21) Anmeldenummer: 87902042.8
(22) Anmeldetag: 18.03.1987
(51) Int. Cl.: A61K 31/44, A61K 31/40

(54) **NIFEDIPINKOMBINATIONSPRÄPARAT**
NIFEDIPINE COMBINATION PREPARATION
PREPARATION COMBINEE DE NIFEDIPINE

(30) Priorität: 21.03.1986 DE 3610037
(43) Veröffentlichungstag der Anmeldung: 30.03.1988
(73) Patentinhaber: SCHERING AKTIENGESELLSCHAFT, 13342 Berlin (DE)
(72) Erfinder: TACK, Johannes-Wilhelm, D-1000 Berlin 20 (DE); ALBRING, Manfred, D-1000 Berlin 27 (DE); WINDT-HANKE, Fred, D-1000 Berlin 26 (DE)
(86) Internationale Anmeldenummer: DE8700113
(87) Internationale Veröffentlichungsnummer: WO8705511

(56) Entgegenhaltungen:
- EP-A- 0 142 561
- EP-A- 0 165 450
- EP-A- 0 169 470
- GB-A- 2 084 017
- The Merck Index, Auflage 10, 1983 Merck & Co., Inc., (Rahway, New Jersey, USA), siehe Seiten 835-6, Nr. 5684 "Mepindolol"

## Beschreibung

Die Erfindung betrifft Nifedipin-Kombinationspräparate, enthaltend Nifedipin mit verzögerter Wirkstofffreisetzung und einen β-Blocker jeweils in granulierter Form und ihre pharmazeutische Verwendung als Therapeutikum bei cardiovasculären Erkrankungen.

In der deutschen Offenlegungsschrift DE-A-34 19 130 werden bereits zum Stand der Technik gehörende Formulierungen für Nifedipin beschrieben, in denen die Bioverfügbarkeit des schwer löslichen Nifedipins verbessert wurde. Möglichkeiten zur Verbesserung der Bioverfügbarkeit werden außerdem von S.H. Yalkowskay in Drugs and the Pharmaceutical Science 12, 135 (1981) und von J. Polderman in "Formulation and preparation of dosage forms 215 (1977), Elsevier," beschrieben und umfassen das Zermahlen von Wirkstoffpartikeln, Kristallstrukturveränderungen, Netzmittelzugabe oder Bildung von Copräzipitaten, um nur einige zu nennen.

In der europäischen Offenlegungsschrift EP-A-142 561 wird eine Nifedipin-Formulierung mit einer über längere Zeit gleichmäßigen Freisetzung beansprucht, die aus einer schnell freigesetzten Zusammensetzung A und einer verzögert freigesetzten Zusammensetzung B besteht. In beiden Zusammensetzungen sind die Nifedipinpartikel nicht größer als 5 µm.
Die langsame Wirkstofffreisetzung wird durch Überziehen der Nifedipin-haltigen Granulate mit Mischungen von wasserunlöslichen Substanzen (wie Mono-, Di- oder Triglyceride, hydrierte Öle und Ethylcellulose) und Magensaftresistenten Lacksubstanzen bewirkt.

Die DE-OS 33 18 649 nennt eine zweiphasige Nifedipinzubereitung in der die Partikelgröße des eingesetzten Nifedipins 1 bis 10 µm beträgt und die Nifedipinkristalle eine spezifische Oberfläche von 1,0 bis 4,0 m²/g haben.

Die europäische Offenlegungsschrift EP-A-165 450 betrifft ein Kombinationspräparat enthaltend Nifedipin und einen β-Blocker. Bevorzugt als β-Blocker sind Acebutolol, Atenolol, Nadolol, Propanolol, Pindolol und Timolol. Die europäische Offenlegungsschrift EP-A-169470 und die britische Offenlegungsschrift GB-A-2084017 betreffen Kombinationspräparate enthaltend ein Dihydropyridin und einen β-Blocker.

Die vorliegende Erfindung betrifft ein Kombinationspräparat, enthaltend 1 Gewichtsteil Nifedipin und 0,05 bis 0,25 Gewichtsteile Mepindolol und übliche Hilfs- und Trägerstoffe.

Der Partikeldurchmesser des verwendeten Nifedipins ist 10-50 µm, bevorzugt 15-50 µm. Die erfindungsgemäßen Kombinationen werden hergestellt, indem man jeweils getrennt Mepindolol und Nifedipin mit bekannten Hilfs- und Trägerstoffen mit Hilfe eines Feuchtgranulationsverfahrens oder eines Trockenkompaktierverfahren granuliert, die Granulate gegebenenfallS mit Lack überzieht und in Hartgelatinekapseln abfüllt. Als Hilfsstoffe kommen Stärke, modifizierte Stärke, Zellulose, Zellulosederivate, quervernetztes Polyvinyl pyrrolidon (PVPP), Natriumalginat und kolloidales Siliciumdioxid, Gelatine, Glukosesirup, Stärkekleister, Polyethylenglykol, Alginate, Magnesiumstearat, Calciumstearat, Stearinsäure, Paraffin, Talkum, pflanzliche oder tierische Fette, Öle und Wachse in Betracht.

Als Füllstoffe sehen Calciumsulfat, Calciumcarbonat, di- und tribasische Calciumphosphate, Magnesiumcarbonat, Magnesiumhydroxycarbonat, Natriumchlorid, Natrium-, Kalium- und Calcium-zitrate, Tartrate und Succinate, Stärke, modifizierte Stärke, Maisstärke, Cellulose, Cellulosepulver, Hydroxypropylcellulose, Zucker wie Lactose, Saccharose oder Dextrose und Zuckeralkohole wie Mannit oder Sorbit genannt.

Als weitere Hilfs- und Trägerstoffe werden Hydroxypropylmethylcellulosephthalat, Cetylalkohol, Ethylcellulose, Titandioxid, hydriertes Rizinusöl, Polysorbat 80, Natriumlaurylsulfat, Methacrylsäurecopolymer (wie Eudragit S 100) verwendet.

Die kombinierte Anwendung von Nifedipin und Mepindolol bietet gegenüber herkömmlichen Präparaten den Vorteil, daß auch mit einer reduzierten Dosis von Nifedipin und Mepindolol eine 24 STd. Blutdrucksenkung erreicht wird. Die Menge des eingesetzten β-Blockers liegt mit 0,05-0,25 Gewichtsteil/ pro Gewichtsteil Nifedipin deutlich unter der in DE-A-34 19 130 angegebenen Menge von 0,5-10 Gewichtsteile β-Blocker/pro Gewichtsteil Nifedipin.

### Beispiele

### Beispiel 1

a) 25 g Mepindololsulfat werden mit 300 g Calciumcitrat und 5 g Magnesiumstearat in einem Turbula-Mischer etwa 10 Minuten vorgemischt. Die Wirkstoffmischung wird in einem geeigneten Mischer zu einer Mischung aus 315 g Laktose, 315 g Maisstärke und 240 g mikrokristalliner Cellulose gegeben und anschließend mit gereinigtem Wasser zu einer plastischen Masse granuliert. Das feuchte Granulat wird durch ein Sieb mit 1.0 mm ⌀ Siebweite extrudiert und anschließend in einem Spheronizer zu Pellets ausgerundet. Die feuchten Granulen (Pellets) werden zum Beispiel in einem Wirbelschichttrockner getrocknet, bis die Ausgangstemperatur 50°C beträgt. Die Pellets können anschließend mit einem lichtundurchlässigen Lack überzogen werden.
b) 400 g Nifedipin (15-50 µm) werden in einer Lösung von 80 g Polysorbat 80, 16 g Natriumlaurylsulfat, 200 g Ethanol abs. und 300 g demin. Wassser suspendiert. Mit dieser Granulierflüssigkeit wird eine Vormischung aus 1070 g Mannitol, 100 g mikrokristalliner Cellulose, 100 g Hydroxypropylcellulose und 100 g Laktose zu einer plastischen Masse granuliert. Das feuchte Granulat wird duch ein Sieb mit 1.0 mm ⌀ Siebweite extrudiert und anschließend mit einem Spheronizer zu Pellets ausgerundet. Die feuchten Granulen (Pellets) werden zum Beispiel in einem Wirbelschichttrockner getrocknet, bis die Ausgangstemperatur 50°C beträgt. 500 g der trockenen Pellets werden in der Wirbelschicht mit einer Lösung von 60 g Hydroxypropylmethylcellulosepthalat` 3,2 g Cetylalkohol, 600 g Ethanol abs. und 900 g Dichlormethan überzogen. Pellets mit Mepindololsulfat (nach a) und Nifedipin (nach b) werden entsprechend den gewünschten Dosierungen (z.B.: 2,5 mg Mepindololsulfat und 20 mg Nifedipin) in Hartgelatinekapseln abgefüllt.

### Beispiel 2

a) 25 g Mepindololsulfat werden mit 60 g Calciumcitrat und 2,5 g Magnesiumstearat in einem Tur bula-Mischer etwa 10 Minuten vorgemischt. Die Wirkstoffmischung wird in einem geeigneten Mischer zu einem Granulat aus 315 g Laktose, 315 g Maisstärke, 240 g mikrokristalliner Cellulose und 240 g Calciumcitrat zugemischt. Dem Substanzgemisch wird 2,5 g Magnesiumstearat zugefügt und 0,5 Minuten gemischt. Die Preßmasse wird zu Mikrotabletten mit zum Beispiel 3 mm ⌀ verpreßt. Die Mikrotabletten können anschließend mit einem Lack aus Hydroxypropylcellulose, Ethylcellulose, Talkum, Titandioxid und hydriertem Rizinusöl in der Wirbelschicht überzogen werden.
b) 400 g Nifedipin (15-50 µm) werden in einer Lösung von 80 g Polysorbat 80, 16 g Natriumlaurylsulfat, 200 g Ethanol abs. und 300 g demin. Wasser suspendiert. Mit dieser Granulierflüssigkeit wird eine Vormischung aus 1070 g Mannitol, 100 g mikrokristalliner Cellulose, 100 g Hydroxypropylcellulose und 100 g Laktose zu einer plastischen Masse granuliert. Das feuchte Granulat wird durch ein Sieb mit 1.0 mm ⌀ Siebweite extrudiert und anschließend in einem Spheronizer zu Pellets ausgerundet. Die feuchten Granulen (Pellets) werden zum Beispiel in einem Wirbelschichttrockner getrocknet, bis die Ausgangstemperatur 50°C beträgt. 500 g der trockenen Pellets werden in der Wirbelschicht mit einer Lösung von 48 g Hydroxypropylmethylcellulosephthalat, 12 g Methacrylsäurecopolymer(zum Beispiel Eudragit S 100), 3,2 g Cetylalkohol, 600 g Ethanol abs. und 900 g Dichlormethan überzogen. Mikrotabletten mit Mepindololsulfat (nach a) und Pellets mit Nifedipin (nach b) werden entsprechend den gewünschten Dosierungen (z. Beispiel 2,5 mg Mepindololsulfat und 20 mg Nifedipin) in Hartgelatinekapseln abgefüllt.

### Beispiel 3

a) 25 g Mepindololsulfat werden mit 60 g Calciumcitrat und 5,0 g Magnesiumstearat in einem Turbula-Mischer etwa 10 Minuten vorgemischt. Die Wirkstoffmischung wird in einem geeigneten Mischer zu einem Granulat aus 315 g Laktose, 315 g Maisstärke, 240 g mikrokristalliner Cellulose und 240 g Calciumcitrat zugemischt.
b) 440 g Nifedipin (15-50 µm) werden in einer Lösung von 17,5 g Natriumlaurylsulfat, 220 g Ethanol abs. und 380 g demin. Wasser suspendiert. Mit dieser Granulierflüssigkeit wird eine Vormischung aus 1080 g Mannitol, 100 g mikrokristalliner Cellulose, 100 g Hydroxypropylcellulose und 100 g Laktose zu einer plastischen Masse granzliert. Das feuchte Granulat wird durch ein Sieb mit 1.0 mm ∅ Siebweite extrudiert und anschließend in einem Spheronizer zu Pellets ausgerundet. Die feuchten Granulen (Pellets) werden zum Beispiel in einem Wirbelschichttrockner getrocknet, bis die Ausgangstemperatur 50°C beträgt. 500 g der trockenen Pellets werden in der Wirbelschicht mit einer Lösung von 60 g Hydroxypropylmethylcellulosephthalat, 3,2 g Cetylalkohol, 600 Ethanol abs. und 900 g Dichlormethan überzogen. Granulate mit Mepindololsulfat (nach a) und Pellets mit Nifedipin (nach b) werden entsprechend den gewünschten Dosierungen (zum Beispiel 2,5 mg Mepindololsulfat und 20 mg Nifedipin) in Hartgelatinekapseln abgefüllt.

## Patentansprüche

1. Kombinationspräparat,
enthaltend 1 Gewichtsteil Nifedipin und 0,05 bis 0,25 Gewichtsteile Mepindolol und übliche Hilfs- und Trägerstoffe.

2. Kombinationspräparat nach Anspruch 1, dadurch gekennzeichnet, daß der Nifedipin-Bestandteil der Kombination eine verzögerte Wirkstofffreisetzung aufweist.

3. Kombinationspräparat nach Anspruch 1, dadurch gekennzeichnet, daß beide Bestandteile der Kombination als Granulate, vorzugsweise als Pellets, vorliegen.

4. Kombinationspräparat nach Anspruch 1, dadurch gekennzeichnet, daß das granulierte Nifedipin und das granulierte Mepindolol in einer Steckkapsel vorliegen.

## Claims

1. Combination preparation,
comprising 1 part by weight of nifedipine and from 0.05 to 0.25 part by weight of mepindolol and customary auxiliaries and carriers.

2. Combination preparation according to claim 1, characterised in that the nifedipine component of the combination exhibits delayed release of the active ingredient.

3. Combination preparation according to claim 1, characterised in that both components of the combination are in the form of granules, preferably in the form of pellets.

4. Combination preparation according to claim 1, characterised in that the granulated nifedipine and the granulated mepindolol are present in a hard gelatin capsule.

## Revendications

1. Préparation associée comprenant 1 partie en poids de nifédipin et de 0,05 à 0,25 partie en poids de mépindolol, avec des adjuvants et véhicules courants.

2. Préparation selon la revendication 1, caractérisée en ce que son nifédipin a un temps retardé de libération de la matière active.

3. Préparation selon la revendication 1, caractérisée en ce que ses deux constituants sont en granulés, de préférence en pastilles ou boulettes.

4. Préparation selon la revendication 1, caractérisée en ce que le nifédipin et le mépindolol granulés sont dans une capsule.
